# EUROPÄISCHE PATENTSCHRIFT

(11) **EP 2 618 771 B1**
(45) Veröffentlichungstag und Bekanntmachung des Hinweises auf die Patenterteilung: **14.08.2019**
(21) Anmeldenummer: 11810991.7
(22) Anmeldetag: 20.09.2011
(51) Int. Cl.: A61B 50/00

(54) **VERPACKUNG**
PACKAGE
EMBALLAGE

(30) Priorität: 21.09.2010 AT 15642010
(43) Veröffentlichungstag der Anmeldung: 31.07.2013
(73) Patentinhaber: Semperit Aktiengesellschaft Holding, 1031 Wien (AT)
(72) Erfinder: ZÖRNPFENNING, Reinhard, 2620 Neunkirchen (AT)
(74) Vertreter: Burger, Hannes
(86) Internationale Anmeldenummer: PCT/AT2011/050010
(87) Internationale Veröffentlichungsnummer: WO 2012/037591

(56) Entgegenhaltungen:
- WO-A1-2004/043810
- US-A- 3 531 906
- US-A- 4 951 815
- US-A- 5 715 943
- US-A- 5 944 189
- US-A1- 2004 005 100
- US-A1- 2005 006 264

## Beschreibung

Die Erfindung betrifft eine Sterilverpackung in Form einer Flachbeutelverpackung, in der chirurgische Handschuhe enthalten sind, mit einer Umhüllung zur Aufnahme der chirurgischen Handschuhe, wobei die Umhüllung zwei einander gegenüberliegende Längsseitenkanten und orthogonal zu diesen zwei einander gegenüberliegende Breitseitenkanten aufweist und in Seitenbereichen Siegelnähte und in zumindest einem Seitenbereich eine Aufreißlasche aufweist, wobei die Umhüllung im Bereich der oder neben den Siegelnähte(n) zumindest teilweise eine Versteifung aufweist, die als rillenförmige Prägung gebildet ist, sowie ein Verfahren zum Verpacken von chirurgischen Handschuhen in einer Sterilverpackung in Form einer Flachbeutelverpackung, nach dem eine Längsseitenkanten und eine Aufreißlasche aufweisende Umhüllung, die zwei einander gegenüberliegende Längsseitenkanten und orthogonal zu diesen zwei einander gegenüberliegende Breitseitenkanten aufweist, bereitgestellt wird und chirurgischen Handschuhe in die Umhüllung eingebracht werden und danach die Umhüllung unter Ausbildung von Siegelnähten versiegelt wird, und die Umhüllung nach der Versiegelung im Bereich der Siegelnähte zumindest bereichsweise geprägt wird, wobei die Prägung rillenförmig ausgebildet wird.

Medizinische Einwegprodukte für den Chirurgiebedarf werden heute üblicherweise steril verpackt im Handel angeboten. Beispielsweise werden chirurgische Handschuhe, d.h. Operationshandschuhe, in so genannten Folienflachbeutelverpackungen paarweise verpackt. Dazu werden die Handschuhe zwischen zwei Verpackungsfolien gelegt - normalerweise werden die Handschuhe vorher in Papierinnentaschen platziert - und die Verpackungsfolie anschließend heißversiegelt. Zum Öffnen der Verpackungen weisen diese zumindest eine Aufreißlasche auf. Dabei wird die Verbindung in den Siegelnähten gelöst, sodass die Handschuhe zum weiteren Gebrauch zugänglich sind. Dabei tritt jedoch das Problem auf, dass eine Verunreinigung der sterilen Innentasche durch die äußere nicht sterile Folie während des Öffnens der Verpackung nicht ausgeschlossen werden kann.

Die DE 15 36 233 A1 beschreibt eine Verpackung für presterilisierte chirurgische Gummihandschuhe. Diese Verpackung weist eine Umhüllung mit
einem Körper auf, in dem ein Paar Handschuhe aufgenommen werden kann. Weiters weist diese Verpackung eine Klappe für das Herüberfalten über den Körper auf, wobei eine Verlängerung der Klappe so angepasst ist, dass diese zurück über die Klappe gefaltet werden kann, wodurch die Verpackung ohne Gefahr einer Verunreinigung der Handschuhe gehandhabt und geöffnet werden kann, ohne dass ein Berühren mit einem Teil der Klappe erfolgt, die möglicherweise in Berührung mit den Handschuhen kommt, sobald diese aus der Verpackung entfernt werden.

Nachteilig daran ist, dass diese Verpackung relativ kompliziert ist, wodurch es zu einer Verteuerung in der automatischen Handschuhherstellung kommt.

Aus der US 5,944,189 A ist eine Verpackung bekannt, die in automatischen Prozessen besser verarbeitet werden kann. Dazu weist die Verpackung eine stabilisierte Form auf, die möglichst flach bzw. ebenflächig ist. In einer Ausführungsform werden dazu die Seitenränder der Verpackung nach innen gefaltet. Nach einer weiteren Ausführungsvariante ist vorgesehen, im Bereich der längs verlaufenden Siegelnähte eine Rippe auszubilden, indem das Verpackungsmaterial entsprechend umgeformt, d.h. dreimal gefaltet, wird, wodurch eine dreieckförmige Rippe ausgebildet wird. Zum Unterschied zu den "aufgedoppelten" Seitenkanten, können diese Rippen flach gepresst werden, sodass sie lediglich die Dicke der Verpackung erhöhen. Nach einer anderen Ausführungsform der Verpackung ist vorgesehen, dass Streifen an den Verpackungsrändern mitgesiegelt oder angeklebt werden.

Die US 2004/0005100 A1 beschreibt eine Lebensmittelverpackung mit expandierbaren Streifen, um damit das Kollabieren des Innenraums zu vermeiden. Die Streifen sind longitudinal und transversal an der Verpackung angeordnet.

Aus der US 5,715,943 A ist eine wiederverwendbare sterilisierbare Tasche bekannt, die teilweise zwei nebeneinander angeordnete Siegelnähte aufweist.

Die WO 2004/043810 A1 beschreibt eine wiederverschließbare Tasche. Diese kann u.a. Stäbe aus einem weichen oder einem harten Material im Bereich der Taschenöffnung aufweisen, die sich parallel zur Taschenöffnung oder in einem Winkel von 45 ° zur Taschenöffnung erstrecken. Die Stäbe werden eine entsprechende Hülse eingeschoben, um die Tasche zu verschließen.

Aus der US 4,951,815 A ist eine Verpackung für medizinische Handschuhe bekannt, die neben den Handschuhen auch einen über Siegelnähte abgetrennten Bereich für ein Gleitmittel aufweist.

Die US 3,531,906 A beschreibt eine Verpackung für Kaffee oder andere sauerstoffempfindliche Produkte. Die Verpackung weist Heißsiegelnähte entlang der und beabstandet zu den Breitseitenkanten und eine mittig angeordnete Heißsiegelnaht, die sich in Richtung der Längsseitenkanten erstreckend ausgebildet ist, auf. In den Eckbereichen sind Verschlussprägungen angeordnet, um damit einen besseren Schutz gegen Undichtheiten in den Eckbereichen aufgrund von wiederholtem Umbiegen der Verpackung entlang der Breitseitenkanten zu erreichen.

Die Aufgabe der vorliegenden Erfindung liegt darin, eine verbesserte Verpackung für chirurgische Handschuhe zu schaffen.

Diese Aufgabe wird einerseits durch die eingangs genannte Verpackung gelöst, bei der sich die rillenförmige Prägung über die gesamte Länge und in Richtung der Längsseitenkanten erstreckend ausgebildet ist, und andererseits durch das voranstehend genannte Verfahren, nach dem die rillenförmige Prägung sich über die gesamte der Länge und in Richtung der Längsseitenkanten ausgebildet wird.

Von Vorteil ist dabei, durch die Versteifung der Umhüllung verhindert werden kann sich die Umhüllung nach dem Öffnen wieder einrollt. Es kann also damit vermieden werden, dass die Außenseite der Umhüllung mit der sterilen Innentasche und den sterilen Handschuhen in Kontakt gelangen kann.

Die Versteifung ist durch eine Prägung gebildet. Von Vorteil ist dabei, dass damit die bisher verwendeten Verpackungsmaterialien ohne weitere Adaptierung weiter verwendet werden können, wodurch der Investitionsaufwand für die Verbesserung der Verpackung deutlich reduziert werden kann. Darüber hinaus kann diese Prägung aber auch in gewissem Maße zur Verbindung der Umhüllungsteile miteinander im Bereich der Siegelnähte beitragen, sodass die Verbindung über die Siegelnähte hinsichtlich der Sicherheit der Dichtheit unterstützt wird.

Die Prägung ist rillenförmig ausgebildet, womit ihre Herstellung vereinfacht und die Taktzeit verkürzt werden kann, indem die Verpackung mit Hilfe einer Prägewalze in einem Walzenstuhl hergestellt werden kann.

Dabei ist es bevorzugt, wenn das Versteifungselement oder die Prägung zumindest annähernd parallel zu den Siegelnähten angeordnet bzw. ausgebildet ist. Es kann damit der Effekt des Vermeidens des Einrollens der Umhüllung beim Öffnen der Verpackung über die gesamte Öffnungslänge der Verpackung sichergestellt werden, unabhängig davon, wie weit der Benutzer des medizinischen Produktes die Verpackung entlang der Siegelnähte aufreißt.

Bevorzugt besteht die Umhüllung zumindest teilweise aus einer Kunststofffolie, wodurch die Prägung einfacher ausgebildet werden kann, insbesondere wenn die Prägung bei erhöhter Temperatur durchgeführt wird.

Gemäß einer Ausführungsvariante des Verfahrens ist vorgesehen, dass die Umhüllung heiß versiegelt wird. Dies hat neben dem Vorteil einer verbesserten Siegelnaht auch den Vorteil, dass die Prägung ebenfalls einfacher an der Umhüllung ausgebildet werden kann.

Es ist dabei von Vorteil wenn die Prägung unmittelbar nach der Versiegelung, also bei noch warmer Umhüllung, durchgeführt wird, sodass ein nochmaliges Erwärmen der Umhüllung für den Prägevorgang entfallen kann.

Zum besseren Verständnis der Erfindung wird diese anhand der nachfolgenden Figur näher erläutert.

Es zeigt in schematisch vereinfachter Darstellung:
- Fig. 1: eine Verpackung in Draufsicht;
- Fig. 2: eine nicht unter den Schutzbereich fallende Verpackung in Draufsicht.

Einführend sei festgehalten, dass die in der Beschreibung gewählten Lageangaben, wie z.B. oben, unten, seitlich usw. auf die unmittelbar beschriebene sowie dargestellte Figur bezogen und bei einer Lageänderung sinngemäß auf die neue Lage zu übertragen sind.

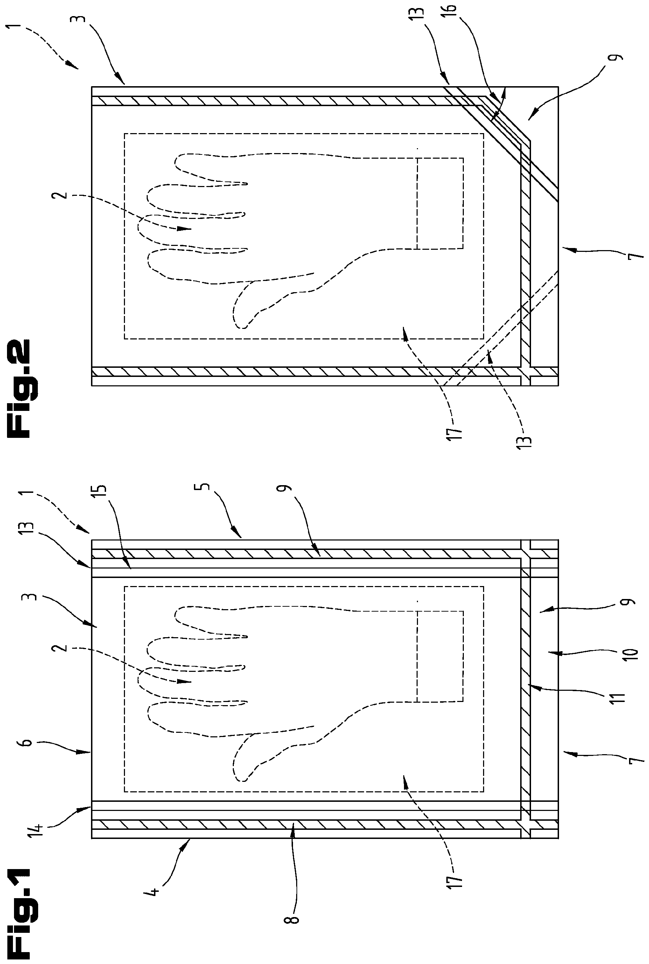

In Fig. 1 ist eine erste Ausführungsvariante einer Verpackung 1 für medizinische Produkte, insbesondere chirurgische Handschuhe 2, in Draufsicht dargestellt. Diese Verpackung 1 wird zum sterilen verpacken des medizinischen Produktes verwendet.

Neben der bevorzugten Verwendung dieser Sterilverpackung für Handschuhe 2 kann diese aber auch zum Verpacken anderer medizinischer Produkte, insbesondere für den Operationsbedarf, verwendet werden, beispielsweise zum Verpacken eines Katheters, von Wundtüchern, von medizinischen Bestecken, von Injektionsnadeln, etc.

Diese Verpackung 1 weist eine Umhüllung 3 auf, die das zu verpackende medizinische Produkt außen umgibt. Diese Umhüllung 3 ist aus den für diesen Zweck bekannten Werkstoffen gebildet. Beispielsweise kann die Umhüllung 3 aus einer Kunststofffolie bestehen, wie z.B. einer Polyethylenfolie, oder auch aus einem medizinischen, nichtfasernden Papier. Zudem kann die Umhüllung 3 auch aus einem Laminat aus verschiedenen Werkstoffen, beispielsweise einer mit einem Papier zumindest teilweise kaschierten Kunststofffolie bestehen. Prinzipiell sind diese Verpackungsmaterialien auf dem Gebiet der Sterilverpackung bekannt, sodass dazu an die einschlägige Literatur verwiesen sei.

Das medizinische Produkt kann presterilisiert in der Verpackung 1 verpackt sein, ebenso besteht die Möglichkeit, dass das medizinische Produkt nach dem Verpacken zusammen mit der Verpackung 1 mit den gängigen Methoden sterilisiert wird, beispielsweise mittels Elektronen- oder Gammastrahlung.

Die Umhüllung 3 weist bei der dargestellten Ausführungsform der Verpackung 1, die als so genannte Flachbeutelverpackung ausgeführt ist, zwei einander gegenüberliegende Längsseitenkanten 4, 5 und orthogonal zu diesen zwei einander gegenüberliegende Breitseitenkanten 6, 7 auf.

Prinzipiell sei jedoch angemerkt, dass die Verpackung 1 nicht zwingend rechteckig - in Draufsicht betrachtet - sein muss, sondern sich deren Geometrie vielmehr nach dem zu verpackenden medizinischen Produkt richtet. Beispielsweise kann die Verpackung 1 auch quadratisch oder zumindest teilweise rund ausgeführt sein.

Zum Verschließen und damit zum sterilen Abdichten des durch die Umhüllung 3 definierten Innenraums der Verpackung sind entlang der Längsseitenkanten 4, 5 jeweils zumindest eine Siegelnaht 8, 9 (Längssiegelnähte), insbesondere Heißsiegelnähte, vorgesehen. Diese Siegelnähte 8, 9 können direkt an den Längsseitenkanten 4, 5 beginnend oder beabstandet zu diesen angeordnet sein. Die Breite der Siegelnähte 8, 9 ist dem Stand der Technik entsprechend ausgeführt.

Des Weiteren sind in der Betrachtungsebene der Fig. 1 einander gegenüberliegend an der unteren Bereitseitenkante 7 der Umhüllung 3 zwei Aufreißlaschen 9, 10 zum Öffnen der Verpackung 1 vorgesehen, an die sich in Richtung auf die obere Breitseitenkante 6 zumindest eine weitere Siegelnaht 11 (Breitensiegelnaht) anschließt. Im Bereich der Aufreißlaschen 9, 10 ist sind die, die Umhüllung 3 bildende obere Teilverpackung und die untere Teilverpackung nicht miteinander verbunden.

Sofern die Umhüllung aus einem einzigen schichtförmigen Material gebildet wird, kann die obere Breitseitenkante 6 der Umhüllung 3 durch eine Falzlinie gebildet sein, sodass sich eine weitere Siegelnaht erübrigt. Es besteht aber auch die Möglichkeit, dass die Umhüllung 3 durch zwei blattförmige Teilverpackungen gebildet wird, sodass in diesem Fall auch an der oberen Breitseitenkante 6 zumindest eine Siegelnaht direkt daran anschließend oder beabstandet zu dieser ausgebildet sein kann.

Es ist vorgesehen, dass die Umhüllung 3 zumindest bereichsweise eine Versteifung aufweist. Diese Versteifung ist als rillenförmige Prägung 13 ausgeführt, beispielsweise in Form von einer oder mehreren, z.B. zwei, drei, vier etc. Längsrillen 14, 15. Diese Längsrillen 14, 15 erstrecken sich über die gesamte Länge der Längsseitenkanten 4, 5 und in Richtung dieser Längsseitenkanten 4, 5. Bevorzugt verlaufen die Längsrillen 14, 15 parallel oder zumindest annähernd parallel zu den oder im Verlauf der Längsseitenkanten 4, 5 und/oder den Siegelnähten 8, 9. Die Prägung 13, also die Längsrillen 14, 15 kann bzw. können beabstandet zu den Siegelnähten 8, 9 angeordnet sein, wobei ein Abstand vorzugsweise gering ist, beispielsweise zwischen 10 % und 200 % der Breite der einer Siegelnaht 8, 9 betragen kann. Alternativ oder zusätzlich dazu kann die oder können die Prägung(en) 13, insbesondere Längsrillen 14, 15, im Bereich der Siegelnähte 8,9 ausgebildet sein.

Bei dieser Ausführung der Versteifung der Umhüllung 3 wird also die Versteifung aus dem Material der Umhüllung 3 selbst gebildet.

Es ist zwar bevorzugt, dass jeweils zumindest eine Prägung entlang der oder in den Siegelnähten 14, 15 angeordnet ist, allerdings besteht auch die Möglichkeit, dass nur eine Versteifung angeordnet wird.

In Fig. 2 ist eine Verpackung 1 gezeigt, wobei wiederum für gleiche Teile gleiche Bezugszeichen bzw. Bauteilbezeichnungen wie in Fig. 1 verwendet werden. Um unnötige Wiederholungen zu vermeiden, wird auf die detaillierte Beschreibung in der vorangegangenen Fig. 1 hingewiesen bzw. Bezug genommen.

Wesentlicher Unterschied zur Ausführungsvariante der Verpackung nach Fig. 1 ist, dass die Versteifung im Bereich der Aufreißlasche 9, insbesondere die rillenförmige Prägung 13, nicht parallel bzw. annähernd parallel bzw. im Verlauf der Siegelnähte 14, 15 sondern winkelig zu diesen angeordnet ist. Ein Winkel 16, den die Versteifung mit der Längsseitenkante 5 einschließt, kann dabei ausgewählt sein aus einem Bereich mit einer unteren Grenze von 20 ° und einer oberen Grenze von 70 °. Bevorzugt wird ein Winkel 16 von 45 °.

Diese nicht unter den Schutzbereich fallende Ausführungsvariante wird insbesondere für Verpackungen 1 mit einseitiger Aufreißlasche 9 verwendet, also bei Ausführungsformen der Verpackung, bei denen die Aufreißlasche 9 nicht wie bei voranstehend beschriebener Ausführungsvariante parallel zu der unteren Breitseitenkante 7 verlaufend ausgebildet ist, sondern im Eckbereich zwischen der Längsseitenkante 5 und der unteren Breitseitenkante 7. Der Eckbereich ist dabei ebenfalls mit einer zusätzlichen Siegelnaht versehen. Im Wesentlichen kann also die Verpackung 1 in einem Winkel zu Längsseitenkante 5 und zur unteren Breitseitenkante 7 geöffnet werden.

Wie strichliert in Fig. 2 angedeutet kann die Umhüllung 3 zwei Versteifungen im Bereich der Aufreißlasche 9 aufweisen, die insbesondere gegengleich gegen die untere Breitseitenkante 7 geneigt sind.

Der Vollständigkeit halber sei angemerkt, dass der Handschuh 2 bei sämtlichen Ausführungsvarianten der Erfindung noch gesondert von einer sterilen Innentasche 17 aufgenommen sein kann, wie dies im Bereich der Sterilverpackung von Handschuhen 2 üblich ist. Diese Innentasche 17 besteht insbesondere aus einem faserfreien Papier.

Des Weiteren sei erwähnt, dass die Handschuhe 2 üblicherweise paarweise und gestülpt verpackt werden.

Die Prägung 13, d.h. die Längsrillung 14, 15, wird bevorzugt unmittelbar nach dem Heißversiegeln der Umhüllung 3 angebracht, wenn die Umhüllung noch warm ist. Bevorzugt wird die Umhüllung 3 dazu zwischen einer Prägewalze, insbesondere aus Stahl, und einer Gummiwalz oder einer gummibeschichteten Walze hindurchgeführt. Es können aber auch andere Prägewerkzeuge verwendet werden.

Gemäß einer weiteren Ausführungsvariante der Erfindung wird die Festigkeit der Siegelnähte 14, 15 auf einen Wert von maximal 3 N pro 15 mm Siegelbreite eingestellt, wodurch die Einrollneigung der Umhüllung während des Peelens der Verpackung beim Öffnen ebenfalls reduziert werden kann. Die Festigkeit wird dabei über den Anpressdruck des Siegelwerkzeuges eingestellt.

Der Ordnung halber sei abschließend darauf hingewiesen, dass zum besseren Verständnis des Aufbaus der Verpackung 1 diese bzw. deren Bestandteile teilweise unmaßstäblich und/oder vergrößert und/oder verkleinert dargestellt wurden.

### Bezugszeichenaufstellung

- 1: Verpackung
- 2: Handschuh
- 3: Umhüllung
- 4: Längsseitenkante
- 5: Längsseitenkante

- 6: Breitseitenkante
- 7: Breitseitenkante
- 8: Siegelnaht
- 9: Siegelnaht
- 10: Aufreißlasche

- 11: Aufreißlasche
- 12: Siegelnaht
- 13: Prägung
- 14: Längsrille
- 15: Längsrille

- 16: Winkel
- 17: Innentasche

## Patentansprüche

1. Sterilverpackung in Form einer Flachbeutelverpackung, in der chirurgische Handschuhe (2) enthalten sind, mit einer Umhüllung (3) zur Aufnahme der chirurgischen Handschuhe (2), wobei die Umhüllung (3) zwei einander gegenüberliegende Längsseitenkanten (4, 5) und orthogonal zu diesen zwei einander gegenüberliegende Breitseitenkanten (6, 7) aufweist und in Seitenbereichen Siegelnähte (8, 9) und in zumindest einem Seitenbereich eine Aufreißlasche (9, 10) aufweist, wobei die Umhüllung im Bereich der oder neben den Siegelnähte(n) (8, 9) zumindest teilweise eine Versteifung aufweist, wobei die Versteifung als rillenförmige Prägung (13,14,15) gebildet ist, **dadurch gekennzeichnet, dass** sich die rillenförmige Prägung (13,14,15) über die gesamte Länge und in Richtung der Längsseitenkanten (4, 5) erstreckend ausgebildet ist.

2. Verpackung (1) nach Anspruch 1, **dadurch gekennzeichnet, dass** die Prägung (13) zumindest annähernd parallel zu den Siegelnähten (8, 9) angeordnet bzw. ausgebildet ist.

3. Verpackung (1) nach einem der 1 oder 2, **dadurch gekennzeichnet, dass** die Umhüllung (3) zumindest teilweise aus einer Kunststofffolie besteht.

4. Verfahren zum Verpacken von chirurgischen Handschuhen (2) in einer Sterilverpackung in Form einer Flachbeutelverpackung, nach dem eine Längsseitenkanten (4, 5) und eine Aufreißlasche (9, 10) aufweisende Umhüllung (3), die zwei einander gegenüberliegende Längsseitenkanten (4, 5) und orthogonal zu diesen zwei einander gegenüberliegende Breitseitenkanten (6, 7) aufweist, bereitgestellt wird und chirurgischen Handschuhe (2) in die Umhüllung (3) eingebracht werden und danach die Umhüllung (3) unter Ausbildung von Siegelnähten (8, 9, 11) versiegelt wird, und die Umhüllung (3) nach der Versiegelung im Bereich der Siegelnähte (8, 9) zumindest bereichsweise geprägt wird, wobei die Prägung (13,14,15) rillenförmig ausgebildet wird, **dadurch gekennzeichnet, dass** die rillenförmige Prägung (13,14,15) über die gesamte Länge und in Richtung der Längsseitenkanten (4, 5) erstreckend ausgebildet wird.

5. Verfahren nach Anspruch 4, **dadurch gekennzeichnet, dass** die Umhüllung (3) heiß versiegelt wird.

6. Verfahren nach Anspruch 4 oder 5, **dadurch gekennzeichnet, dass** die Prägung unmittelbar nach der Versiegelung durchgeführt wird.

## Claims

1. A sterile package in the form of a peel pouch package in which surgical gloves (2) are contained, with a wrapping (3) for receiving the surgical gloves (2), wherein the wrapping (3) comprises two long side edges (4, 5) opposing one another and orthogonally to these two short side edges (6, 7) opposing one another and comprises sealing seams (8, 9) in side areas and a tear-open tap (9, 10) in at least one side area, wherein the wrapping at least partially comprises a stiffening in the area of or next to the sealing seam(s) (8, 9), wherein the stiffening is formed as a groove-shaped embossing (13, 14, 15), **characterized in that** the groove-shaped embossing (13, 14, 15) is formed to extend across the entire length and in the direction of the long side edges (4, 5).

2. The package (1) according to claim 1, **characterized in that** the embossing (13) is arranged or formed at least approximately in parallel to the sealing seams (8, 9).

3. The package (1) according to one of claims 1 or 2, **characterized in that** the wrapping (3) at least partially consists of a plastic film.

4. A method for packaging surgical gloves (2) in a sterile package in the form of a peel pouch package, according to which a wrapping (3) with long side edges (4, 5) and a tear-open tap (9, 10), comprising two long side edges (4, 5) opposing one another and orthogonally to these two short side edges (6, 7) opposing one another, is provided and surgical gloves (2) are inserted into the wrapping (3) and subsequently, the wrapping (3) is sealed under formation of sealing seams (8, 9, 11), and the wrapping (3) is at least in some areas embossed after the sealing in the area of the sealing seams (8, 9), wherein the embossing (13, 14, 15) is formed to be groove-shaped, **characterized in that** the groove-shaped embossing (13, 14, 15) is formed to extend across the entire length and in the direction of the long side edges (4, 5).

5. The method according to claim 4, **characterized in that** the wrapping (3) is heat-sealed.

6. The method according to claim 4 or 5, **characterized in that** the embossing is carried out immediately after the sealing.

## Revendications

1. Emballage stérile sous la forme d'un emballage en sachet plat, dans lequel sont contenus des gants (2) chirurgicaux, avec une enveloppe (3) destinée à recevoir les gants (2) chirurgicaux, l'enveloppe (3) comportant deux arêtes côté longueur (4, 5) opposées et, à angle droit avec celles-ci, deux arêtes côté largeur (6, 7) opposées, et comportant des cordons de scellement (8, 9) dans des zones latérales, et une patte d'ouverture (9, 10) dans au moins une zone latérale, l'enveloppe comportant au moins partiellement un renfort dans la zone des cordons de scellement (8, 9) ou à proximité de ceux-ci, le renfort étant formé en tant que gaufrage en forme de rainures (13, 14, 15), **caractérisé en ce que** le gaufrage en forme de rainures (13, 14, 15) est constitué en s'étendant sur toute la longueur et en direction des arêtes côté longueur (4, 5).

2. Emballage (1) selon la revendication 1, **caractérisé en ce que** le gaufrage (13) est disposé ou respectivement constitué au moins à peu près parallèlement aux cordons de scellement (8, 9).

3. Emballage (1) selon la revendication 1 ou 2, **caractérisé en ce que** l'enveloppe (3) est composée au moins partiellement d'une feuille de matière plastique.

4. Procédé d'emballage de gants (2) chirurgicaux dans un emballage stérile sous la forme d'un emballage en sachet plat, selon lequel est fournie une enveloppe (3) qui comporte des arêtes côté longueur (4, 5) et une patte d'ouverture (9, 10) et qui comporte deux arêtes côté longueur (4, 5) opposées et, à angle droit avec celles-ci, deux arêtes côté largeur (6, 7) opposées, et des gants (2) chirurgicaux sont mis en place dans l'enveloppe (3), et ensuite l'enveloppe (3) est scellée avec constitution de cordons de scellement (8, 9, 11) et, après le scellement, l'enveloppe (3) est gaufrée au moins par tronçons dans la zone des cordons de scellement (8, 9), le gaufrage (13, 14, 15) étant constitué en forme de rainures, **caractérisé en ce que** le gaufrage (13, 14, 15) en forme de rainures est constitué en s'étendant sur toute la longueur et en direction des arêtes côté longueur (4, 5).

5. Procédé selon la revendication 4, **caractérisé en ce que** l'enveloppe (3) est scellée à chaud.

6. Procédé selon la revendication 4 ou 5, **caractérisé en ce que** le gaufrage est effectué immédiatement après le scellement.
